# EUROPEAN PATENT SPECIFICATION

(11) **EP 2 949 362 B1**
(45) Date of publication and mention of the grant of the patent: **16.11.2022**
(21) Application number: 15169179.7
(22) Date of filing: 26.05.2015
(51) Int. Cl.: A61K 8/9761, A61K 8/9789, A61K 8/9794, A61Q 19/08, A61P 3/10, A61P 17/00, A61P 9/10, A61P 19/10, A61P 25/28, A61P 27/12, A61P 29/00, A61P 39/06, A61K 36/28, A61K 36/36, A61K 36/48, A61K 36/534, A61K 36/535, A61K 36/704, A61K 36/708, A61K 36/53

(54) **AGES-DEGRADING AGENT AND USE THEREOF**
MITTEL ZUR ALTERSHERABSETZUNG UND VERWENDUNG DAVON
AGENT DÉGRADANT D'ÂGE ET SON UTILISATION

(30) Priority: 26.05.2014 JP 2014108121; 21.05.2015 JP 2015104009
(43) Date of publication of application: 02.12.2015
(73) Proprietor: ARKRAY, Inc., Minami-ku Kyoto-shi Kyoto 601-8045 (JP); The Doshisha, Kyoto 602-8580 (JP)
(72) Inventor: Yagi, Masayuki, Kyoto, 610-0394 (JP); Yonei, Yoshikazu, Kyoto, 610-0394 (JP); Kawai, Hiroshige, Kyoto, 602-0008 (JP); Shoshihara, Masako, Kyoto, 602-0008 (JP)
(74) Representative: Hoffmann Eitle

(56) References cited:
- EP-A1- 2 939 657
- JP-A- 2004 002 237
- JP-A- 2004 250 445
- JP-A- 2010 248 150
- US-A1- 2012 207 862
- DATABASE GNPD [Online] MINTEL; October 2003 (2003-10), "Tightenin Lift Botanical Serum", XP002744686, Database accession no. 2175113

## Description

### BACKGROUND

### Field of the Invention

The present invention relates to AGEs-degrading agents and use thereof.

### Description of the Related Art

It is known that oxidation reactions and glycation reactions in vivo adversely affect cells and tissues. In particular, the production and accumulation of proteins having undergone an oxidation reaction or a glycation reaction, i.e. oxidized proteins or glycated proteins, cause diseases such as diabetes complications, Alzheimer's disease, cataracts, and arteriosclerosis, as well as aging and hypofunction of tissues such as skin. On this account, the oxidized proteins and glycated proteins are called damaging proteins. In particular, accumulation of AGEs (Advanced Glycation End-products), which are end products produced by a non-enzymatic addition reaction of a carbohydrate to a protein, is seen as a problem.

Normally, these damaging proteins are degraded and removed by proteasomes and proteases such as oxidized protein hydrolase (hereinafter referred to as "OPH"). However, the activities of these enzymes are deteriorated with age. Thus, expectations are rising regarding the possibility of treating and preventing the above-described diseases, aging of tissues, and hypofunction of tissues by enhancing the activities of these enzymes.

Proteasomes are large enzyme complexes that degrade proteins. Examples of the proteasomes include 26S proteasome and 20S proteasome. Known as substances that activate the proteasomes are soybean saponin, kale extract, silybin, silane compounds, iris extract, and the like (Patent Documents 1 to 5). OPH is a serine protease. As substances that activate OPH, Roman chamomile (*Anthemis nobilis L.),* chameleon plant (*Houttuynia cordata Thunberg*)*,* whitethorn (*Crataegus oxyacantha L*.)*,* and grapes (*Vitis vinifera L*.) are known (Patent Document 6). However, in recent years, there are increasing demands for novel degrading agents that can degrade AGEs efficiently.

JP 2004 250445 relates to a glycation inhibitor having high glycation inhibitory effects without causing side effects comprising at least one kind of extract of a plant selected from different types of plants.

JP 2010 248150 relates to an agent for preventing, inhibiting or ameliorating skin deterioration stemming from AGEs buildup containing (a) lactic acid, glycolic acid, salicylic acid, malic acid, citric acid, phytic acid and at least one selected from the group consisting of a pharmaceutically-permitted salt thereof, and (b) an advanced glycation end products decomposition agent as an active ingredient.

Entry 2175113 of database GNPD [online] Mintel, October 2003, relates to a tightening lift botanical serum.

JP 2004/002237 A1 relates to an anti-aging food or medicine for the humans and or animals and is characterized by containing the herb and/or its treated product which contain one or more herb ingredients selected from the group consisting of phenylpropanoid, monoterpenoid,sesquiterpenoid, triterpenoid, coumarin, isocoumarin, chroman, TAROKIN, tannin and lignan-based ingredients.

US 2012/0207862 A1 relates to compositions and agents for blocking serum and other aging factors, especially arNOX in serum, skin or other body fluid or tissue and/or on the cell surface, and methods for using the same.

### Citation List

### Patent Document(s)

- Patent Document 1: JP 2002-179592 A
- Patent Document 2: Japanese Patent No. 4255432
- Patent Document 3: JP 2008-308505 A
- Patent Document 4: JP 2004-91398 A
- Patent Document 5: JP 2007-99650 A
- Patent Document 6: WO 2011/004733

### SUMMARY

### Problem to be Solved by the Invention

With the foregoing in mind, the present invention provides a non-therapeutic anti-aging method, a formulation comprising an AGEs-degrading agent for therapeutic use and a method for degrading AGEs in a sample as defined by the claims.

### Means for Solving Problem

The present invention uses an AGEs-degrading agent containing at least one kind of plant extract obtained from a plant selected from the group consisting of peanut, knotweed and leaf lettuce.

The present disclosure also provides an anti-aging agent containing the AGEs-degrading agent of the present invention.

The present invention also provides a formulation for use in preventing or treating diseases, and hypofunction of tissues caused by AGEs, containing the AGEs-degrading agent as defined by the claims.

The present invention also provides a method for degrading AGEs, including the step of: degrading AGEs in a sample by adding the AGEs-degrading agent as defined by the claims to the sample.

The present invention also provides a non-therapeutic anti-aging method including the stape of: administering the AGEs-degrading agent as defined by the claims.

The present disclosure also provides a method for preventing or treating diseases, aging of tissues, and hypofunction of tissues caused by AGEs, including the step of: administering the AGEs-degrading agent used in the present disclosure or the anti-aging agent of the present disclosure to a living organism.

### Effects of the Invention

According to the AGEs-degrading agent used in the present invention, it is possible to degrade AGEs. Therefore, the present disclosure can treat and prevent diseases, aging of tissues, and hypofunction of tissues caused by AGEs produced in vivo, for example. Furthermore, the present invention is excellent in terms of safety, because it uses plant extracts that have been used since ancient times. Also, the present invention is excellent in terms of productivity, because the plant extracts can be obtained in large quantities.

### DETAILED DESCRIPTION

### A GEs-degrading agent and AGEs-degrading method

As described above, the AGEs-degrading agent used in the present invention contains at least one kind of plant extract obtained from a plant selected from the group consisting of peanut, knotweed and leaf lettuce.

The AGEs-degrading agent may contain one of the above-described plant extracts or two or more of the above-described plant extracts, for example. These extracts may not only have AGEs-degrading ability but also have, for example, activity similar to that of oxidized protein hydrolase (OPH) or OPH activity-enhancing ability.

The peanut (scientific name: *Arachis hypogaea*) is a plant belonging to the genus *Arachis* of the family Fabaceae. An extract of the peanut may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the peanut, the part from which the extract is obtained is not particularly limited, and the extract of the peanut preferably is a seed extract, for example.

The rosemary (scientific name: *Rosmarinus officinalis)* is a plant belonging to the genus *Rosmarinus* of the family Lamiaceae. An extract of the rosemary may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the rosemary, the part from which the extract is obtained is not particularly limited, and the extract may be a leaf extract, for example.

The knotweed (scientific name: *Persicaria hydropiper)* is a plant belonging to the genus *Persicaria* of the family Polygonaceae. Examples of the knotweed include water pepper and *Polygonum hydropiperf purpurascens*, which is a variant species. An extract of the knotweed may be obtained from any of its flower, spike, ear, pericarp, fruit, stem, branch, leaf, rhizome, root bark, root, seed, plumule, and the like, for example. The extract may be obtained from one part or from two or more parts. Alternatively, the extract may be obtained from the entire plant. In the extract of the knotweed, the part from which the extract is obtained is not particularly limited, and the extract may be a plumule extract, for example.

The leaf lettuce (scientific name: *Lactuca sativa var. crispa*) is a plant belonging to the genus *Lactuca* of the family Asteraceae. An extract of the leaf lettuce may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the leaf lettuce, the part from which the extract is obtained is not particularly limited, and the extract may be a leaf extract, for example.

The basil (scientific name: *Ocimum basilicum)* is a plant belonging to the genus *Ocimum* of the family Lamiaceae. The basil is a plant often used in, for instance, Italian food etc., and also is known by the name of "basilico", "basil", and "sweet basil". An extract of the basil may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the basil, the part from which the extract is obtained is not particularly limited, and the extract may be a leaf extract, for example.

The thyme (scientific name: *Thymus vulgaris)* is a plant belonging to the genus *Thymus* of the family Lamiaceae. An extract of the thyme may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the thyme, the part from which the extract is obtained is not particularly limited, and the extract may be a leaf extract, for example.

The spearmint (scientific name: *Mentha spicata*) is a plant belonging to the genus *Mentha* of the family Lamiaceae. An extract of the spearmint may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the spearmint, the part from which the extract is obtained is not particularly limited, and the extract may be a leaf extract, for example.

The lemon balm (scientific name: *Melissa officinalis)* is a plant belonging to the genus *Melissa* of the family Lamiaceae. An extract of the lemon balm may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the lemon balm, the part from which the extract is obtained is not particularly limited, and the extract may be a leaf extract, for example.

The perilla (scientific name: *Perilla frutescens*) is a plant belonging to the genus *Perilla* of the family Lamiaceae. Examples of the perilla include wild species and variant species of wild sesame *(Perilla frutescens* var. *frutescens).* An extract of the perilla may be obtained from any of its flower, spike, ear, pericarp, fruit, stem, branch, leaf, rhizome, root bark, root, seed, inflorescence of perilla (young spike of perilla), and the like, for example. The extract may be obtained from one part or from two or more parts. Alternatively, the extract may be obtained from the entire plant. In the extract of the perilla, the part from which the extract is obtained is not particularly limited, and the extract may be a spike extract, for example. Specific examples of the spike extract include an extract of inflorescence of perilla (young spike of perilla).

The rhubarb (scientific name: *Rheum rhabarbarum)* is a plant belonging to the genus *Rheum* of the family Polygonaceae. An extract of the rhubarb may be obtained from any of its flower, spike, ear, pericarp, fruit, stem, branch, leaf, rhizome, root bark, root, seed, petiole, and the like, for example. The extract may be obtained from one part or from two or more parts. Alternatively, the extract may be obtained from the entire plant. In the extract of the rhubarb, the part from which the extract is obtained is not particularly limited, and the extract may be a petiole extract, for example.

The AGEs-degrading agent used in the present invention preferably has not only high AGEs-degrading ability but also high oxidized protein hydrolase (OPH)-activating ability, for example. If the AGEs-degrading agent used in the present invention has the AGEs-degrading ability and the OPH-activating ability, the AGEs-degrading agent can degrade more kinds of AGEs and a larger amount of AGEs, for example. Among the above-described extracts, extracts of peanut, rosemary, knotweed, leaf lettuce, basil, thyme, spearmint, lemon balm, perilla, and rhubarb have high AGEs-degrading ability. Thus, for the AGEs-degrading ability, the AGEs-degrading agent used in the present invention contains at least one kind of plant extract obtained from a plant selected from the group consisting of peanut, knotweed and leaf lettuce, preferably at least one kind of plant extract obtained from a plant selected from the group consisting of knotweed. Furthermore, among the above-described extracts, extracts of rosemary, basil, thyme, spearmint and lemon balm have OPH activity-enhancing ability, in addition to the AGEs-degrading ability. Thus, for example, for the OPH activity-enhancing ability, the AGEs-degrading agent of the present disclosure preferably contains, among the above-described extracts, extracts that also have OPH activity-enhancing ability. In the case where not only AGEs-degrading ability but also OPH activity-enhancing ability is to be imparted to the AGEs-degrading agent of the present disclosure the combination of the extracts is not particularly limited. The combination may be, for example, the combination of rosemary, thyme, and spearmint.

When the AGEs-degrading agent used in the present invention contains two or more kinds of plant extracts, it is preferable that the AGEs-degrading agent contains, as the two or more kinds of plant extracts: at least one kind of plant extract obtained from a plant selected from the group consisting of peanut, knotweed, leaf lettuce, perilla, and rhubarb; and at least one kind of plant extract obtained from a plant selected from the group consisting of rosemary, basil, thyme, spearmint and lemon balm, which also serve to enhance the OPH activity, for example.

The AGEs-degrading agent used in the present invention further may contain, in addition to the at least one kind of plant extract obtained from a plant selected from the group consisting of peanut, knotweed and leaf lettuce, a plant extract(s) other than the above plant extracts, for example.

As the above-described other plant extract, the AGEs-degrading agent used in the present invention may contain an OPH activity enhancer, for example. Examples of the activity enhancer include extracts of water chestnut, stevia, chrysanthemum, savory, mugwort, and chestnut. Among the above extracts, the OPH activity enhancer may contain one kind of plant extract or two or more kinds of plant extracts, for example.

The water chestnut (scientific name: *Trapa japonica*) is a plant belonging to the genus *Trapa* of the family Trapaceae. An extract of the water chestnut may be obtained from any of its flower, spike, ear, pericarp, fruit, stem, branch, leaf, rhizome, root bark, root, seed, seed coat, and the like, for example. The extract may be obtained from one part or from two or more parts. Alternatively, the extract may be obtained from the entire plant, for example. In the extract of the water chestnut, the part from which the extract is obtained is not particularly limited, and the extract may be a seed coat extract, for example.

The stevia (scientific name: *Stevia rebaudiana*) is a plant belonging to the genus *Stevia* of the family Asteraceae. An extract of the stevia may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the stevia, the part from which the extract is obtained is not particularly limited, and the extract may be a leaf extract, for example.

The chrysanthemum (scientific name: *Chrysanthemum morifolium)* is a plant belonging to the genus *Chrysanthemum* of the family Asteraceae. An extract of the chrysanthemum may be obtained from any of its flower, spike, ear, pericarp, fruit, stem, branch, leaf, rhizome, root bark, root, seed, petal, and the like, for example. The extract may be obtained from one part or from two or more parts. Alternatively, the extract may be obtained from the entire plant. In the extract of the chrysanthemum, the part from which the extract is obtained is not particularly limited. The extract of the chrysanthemum may be, for example, a petal extract, and preferably is a petal extract obtained from an edible chrysanthemum.

The savory (scientific name: *Satureia hortensis* (for summer savory) and *Satureia montana* (for winter savory)) is a plant belonging to the genus *Satureja* of the family Lamiaceae. An extract of the savory may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the savory, the part from which the extract is obtained is not particularly limited, and the extract may be a leaf extract or a spike extract, for example.

The mugwort (scientific name: *Artemisia princeps*) is a plant belonging to the genus *Artemisia* of the family Asteraceae. An extract of the mugwort may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the mugwort, the part from which the extract is obtained is not particularly limited, and the extract may be a leaf extract, for example.

The chestnut (scientific name: *Castanea crenata*) is a plant belonging to the genus *Castanea* of the family Fagaceae. An extract of the chestnut may be obtained from any of its flower, spike, ear, pericarp, fruit, stem, branch, leaf, rhizome, root bark, root, seed, outer skin, astringent skin, and the like, for example. The extract may be obtained from one part or from two or more parts. Alternatively, the extract may be obtained from the entire plant. In the extract of the chestnut, the part from which the extract is obtained is not particularly limited, and the extract may be, for example, an extract obtained from outer skin or astringent skin.

Also, as the above-described other plant extract, the AGEs-degrading agent used in the present invention may contain an antioxidant having antioxidative activity, for example. When the AGEs-degrading agent used in the present invention further contains the antioxidant, the AGEs-degrading agent can further inhibit the acceleration of AGEs production caused by oxidative stress other than glycation reactions, for example. Examples of the antioxidant include extracts of lemon balm, spearmint, savory, hibiscus, rosemary, thyme, clove, basil, peppermint, marjoram, oregano, tarragon, bay leaf, star anise, paprika, allspice, garlic, cardamon, onion, cinnamon, blue mallow, Japanese pepper, anise, lemongrass, chili peppers such as red chili and green chili, juniper berry, ajwain, caraway, turmeric, fennel, dill, black cumin, celery, fenugreek, coriander, peppers (e.g., black pepper, white pepper, and red pepper), cumin, and ginger. Among the above extracts, the antioxidant may contain one kind of plant extract or two or more kinds of plant extracts, for example.

The lemon balm (scientific name: *Melissa officinalis*) is a plant belonging to the genus *Melissa* of the family Lamiaceae. An extract of the lemon balm may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the lemon balm, the part from which the extract is obtained is not particularly limited, and the extract may be a leaf extract, for example.

The spearmint (scientific name: *Mentha spicata*) is a plant belonging to the genus *Mentha* of the family Lamiaceae. An extract of the spearmint may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the spearmint, the part from which the extract is obtained is not particularly limited, and the extract may be a leaf extract, for example.

The savory (scientific name: *Satureia hortensis* (for summer savory) and *Satureia montana* (for winter savory)) is a plant belonging to the genus *Satureja* of the family Lamiaceae. An extract of the savory may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the savory, the part from which the extract is obtained is not particularly limited, and the extract may be a leaf extract, for example.

The hibiscus (scientific name: *Hibiscus sabdariffa*) is a plant belonging to the genus Hibiscus of the family Malvaceae. An extract of the hibiscus may be obtained from any of its flower, spike, ear, pericarp, fruit, stem, branch, leaf, rhizome, root bark, root, seed, calyx, bract, and the like, for example. The extract may be obtained from one part or from two or more parts. Alternatively, the extract may be obtained from the entire plant. In the extract of the hibiscus, the part from which the extract is obtained is not particularly limited, and the extract may be an extract obtained from grown calyx and/or grown bract, for example.

The rosemary (scientific name: *Rosmarinus officinalis*) is a plant belonging to the genus *Rosmarinus* of the family Lamiaceae. An extract of the rosemary may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the rosemary, the part from which the extract is obtained is not particularly limited, and the extract may be a leaf extract, for example.

The thyme (scientific name: *Thymus vulgaris*) is a plant belonging to the genus *Thymus* of the family Lamiaceae. An extract of the thyme may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the thyme, the part from which the extract is obtained is not particularly limited, and the extract may be a leaf extract, for example.

The clove (scientific name: *Syzygium aromaticum*) is a plant belonging to the genus *Syzygium* of the family Myrtaceae. An extract of the clove may be obtained from any of its flower, spike, ear, pericarp, fruit, stem, branch, leaf, rhizome, root bark, root, seed, flower bud, and the like, for example. The extract may be obtained from one part or from two or more parts. Alternatively, the extract may be obtained from the entire plant. In the extract of the clove, the part from which the extract is obtained is not particularly limited, and the extract may be a flower bud extract, for example.

The basil (scientific name: Ocimum basilicum) is a plant belonging to the genus *Ocimum* of the family Lamiaceae. An extract of the basil may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the basil, the part from which the extract is obtained is not particularly limited, and the extract may be a leaf extract, for example.

The peppermint (scientific name: *Mentha piperita*) is a plant belonging to the genus *Mentha* of the family Lamiaceae. An extract of the peppermint may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the peppermint, the part from which the extract is obtained is not particularly limited, and the extract may be a leaf extract, for example.

The marjoram (scientific name: *Origanum majorana*) is a plant belonging to the genus *Origanum* of the family Lamiaceae. An extract of the marjoram may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the marjoram, the part from which the extract is obtained is not particularly limited, and the extract may be a leaf extract, for example.

The oregano (scientific name: *Origanum vulgare)* is a plant belonging to the genus *Origanum* of the family Lamiaceae. An extract of the oregano may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the oregano, the part from which the extract is obtained is not particularly limited, and the extract may be a leaf extract, for example.

The tarragon (scientific name: *Artemisia dracunculus*) is a plant belonging to the genus *Artemisia* of the family Asteraceae. An extract of the tarragon may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the tarragon, the part from which the extract is obtained is not particularly limited, and the extract may be a leaf extract, for example.

The bay leaf (scientific name: *Laurus nobilis*) is a plant belonging to the genus *Laurus* of the family Laureaceae. An extract of the bay leaf may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the bay leaf, the part from which the extract is obtained is not particularly limited, and the extract may be a leaf extract, for example.

The star anise (scientific name: *Illicium verum*) is a plant belonging to the genus *Illicium* of the family Schisandraceae. An extract of the star anise may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the star anise, the part from which the extract is obtained is not particularly limited, and the extract may be a fruit extract, for example.

The paprika (scientific name: *Capsicum annuum)* is a plant belonging to the genus *Capsicum* of the family Solanaceae. An extract of the paprika may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the paprika, the part from which the extract is obtained is not particularly limited, and the extract may be a fruit extract, for example.

The allspice (scientific name: *Pimenta dioica*) is a plant belonging to the genus *Pimenta* of the family Myrtaceae. An extract of the allspice may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the allspice, the part from which the extract is obtained is not particularly limited, and the extract may be a fruit extract, for example.

The garlic (scientific name: *Allium sativum)* is a plant belonging to the genus *Allium* of the family Amaryllidaceae. An extract of the garlic may be obtained from any of its flower, spike, ear, pericarp, fruit, stem, branch, leaf, rhizome, root bark, root, seed, bulb, and the like, for example. The extract may be obtained from one part or from two or more parts. Alternatively, the extract may be obtained from the entire plant. In the extract of the garlic, the part from which the extract is obtained is not particularly limited, and the extract may be a bulb extract, for example.

The cardamon (scientific name: *Elettaria cardamomum)* is a plant belonging to the genus *Elettaria* of the family Zingiberaceae. An extract of the cardamon may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the cardamon, the part from which the extract is obtained is not particularly limited, and the extract may be a seed extract, for example.

The onion (scientific name: *Allium cepa*) is a plant belonging to the genus *Allium* of the family Amaryllidaceae. An extract of the onion may be obtained from any of its flower, spike, ear, pericarp, fruit, stem, branch, leaf, rhizome, root bark, root, seed, bulb, and the like, for example. The extract may be obtained from one part or from two or more parts. Alternatively, the extract may be obtained from the entire plant. In the extract of the onion, the part from which the extract is obtained is not particularly limited, and the extract may be a bulb extract, for example.

The cinnamon (scientific name: *Cinnamomum verum)* is a plant belonging to the genus *Cinnamomum* of the family Laureaceae. An extract of the cinnamon may be obtained from any of its flower, spike, ear, pericarp, fruit, stem, branch, leaf, rhizome, root bark, root, seed, bark, and the like, for example. The extract may be obtained from one part or from two or more parts. Alternatively, the extract may be obtained from the entire plant. In the extract of the cinnamon, the part from which the extract is obtained is not particularly limited, and the extract may be a bark extract, for example.

The blue mallow (scientific name: *Malva sylvestris)* is a plant belonging to the genus *Malva* of the family Malvaceae. An extract of the blue mallow may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the blue mallow, the part from which the extract is obtained is not particularly limited, and the extract may be a flower extract, for example.

The Japanese pepper (scientific name: *Zanthoxylum piperitum)* is a plant belonging to the genus *Zanthoxylum* of the family Rutaceae. An extract of the Japanese pepper may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the Japanese pepper, the part from which the extract is obtained is not particularly limited, and the extract may be a fruit extract, for example.

The anise (scientific name: *Pimpinella anisum*) is a plant belonging to the genus *Pimpinella* of the family Apiaceae. An extract of the anise may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the anise, the part from which the extract is obtained is not particularly limited, and the extract may be a fruit extract, for example.

The lemongrass (scientific name: *Cymbopogon citratus*) is a plant belonging to the genus *Cymbopogon* of the family Poaceae. An extract of the lemongrass may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the lemongrass, the part from which the extract is obtained is not particularly limited, and the extract may be a leaf extract, for example.

The chili pepper (scientific name: *Capsicum annuum)* is a plant belonging to the genus *Capsicum* of the family Solanaceae. An extract of the chili pepper may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the chili pepper, the part from which the extract is obtained is not particularly limited, and the extract may be a fruit extract, for example.

The juniper berry (scientific name: *Juniperus communis)* is a plant belonging to the genus *Juniperus* of the family Cupressaceae. An extract of the juniper berry may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the juniper berry, the part from which the extract is obtained is not particularly limited, and the extract may be a fruit extract, for example.

The ajwain (scientific name: *Trachyspermum ammi*) is a plant belonging to the genus *Trachyspermum* of the family Apiaceae. An extract of the ajwain may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the ajwain, the part from which the extract is obtained is not particularly limited, and the extract may be a seed extract, for example.

The caraway (scientific name: *Carum carvi)* is a plant belonging to the genus *Carum* of the family Apiaceae. An extract of the caraway may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the caraway, the part from which the extract is obtained is not particularly limited, and the extract may be a fruit extract, for example.

The turmeric (scientific name: *Curcuma longa)* is a plant belonging to the genus *Curcuma* of the family Zingiberaceae. An extract of the turmeric may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the turmeric, the part from which the extract is obtained is not particularly limited, and the extract may be a rhizome extract, for example.

The fennel (scientific name: *Foeniculum vulgare)* is a plant belonging to the genus *Foeniculum* of the family Apiaceae. An extract of the fennel may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the fennel, the part from which the extract is obtained is not particularly limited, and the extract may be a leaf extract, for example.

The dill (scientific name: *Anethum graveolens*) is a plant belonging to the genus *Anethum* of the family Apiaceae. An extract of the dill may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the dill, the part from which the extract is obtained is not particularly limited, and the extract may be a seed extract, for example.

The black cumin (scientific name: *Nigella satibva*) is a plant belonging to the genus *Nigella* of the family Ranunculaceae. An extract of the black cumin may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the black cumin, the part from which the extract is obtained is not particularly limited, and the extract may be a seed extract, for example.

The celery (scientific name: *Apium graveolens*) is a plant belonging to the genus *Apium* of the family Apiaceae. An extract of the celery may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the celery, the part from which the extract is obtained is not particularly limited, and the extract may be a leaf extract, for example.

The fenugreek (scientific name: *Trigonella foenum-graecum*) is a plant belonging to the genus *Trigonella* of the family Fabaceae. An extract of the fenugreek may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the fenugreek, the part from which the extract is obtained is not particularly limited, and the extract may be a seed extract, for example.

The coriander (scientific name: *Coriandrum sativum*) is a plant belonging to the genus *Coriandrum* of the family Apiaceae. An extract of the coriander may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the coriander, the part from which the extract is obtained is not particularly limited, and the extract may be a leaf extract, for example.

The pepper (scientific name: *Piper nigrum*) is a plant belonging to the genus *Piper* of the family Piperaceae. An extract of the pepper may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the pepper, the part from which the extract is obtained is not particularly limited, and the extract may be a fruit extract, for example.

The cumin (scientific name: *Cuminum cyminum*) is a plant belonging to the genus *Cuminum* of the family Apiaceae. An extract of the cumin may be obtained from any of its flower, spike, ear, pericarp, fruit, stem, branch, leaf, rhizome, root bark, root, seed, and the like, for example. The extract may be obtained from one part or from two or more parts. Alternatively, the extract may be obtained from the entire plant. In the extract of the cumin, the part from which the extract is obtained is not particularly limited, and the extract may be a seed extract, for example.

The ginger (scientific name: *Zingiber officinale*) is a plant belonging to the genus *Zingiber* of the family Zingiberaceae. An extract of the ginger may be obtained from any of its flower, spike, ear, pericarp, fruit, stem, branch, leaf, rhizome, root bark, root, seed, tuberous root, and the like, for example. The extract may be obtained from one part or from two or more parts. Alternatively, the extract may be obtained from the entire plant. In the extract of the ginger, the part from which the extract is obtained is not particularly limited, and the extract may be a seed extract, for example.

In the present invention, the extract can be obtained from any of the above-described parts or from the entire plant, for example. The method for obtaining the extract is not particularly limited, and examples thereof include known methods such as solvent extraction and expression.

An extraction solvent to be used in the solvent extraction is not particularly limited, and examples thereof include aqueous solvents and organic solvents. The aqueous solvents are not particularly limited, and examples thereof include water. The organic solvents are not particularly limited, and examples thereof include lower alcohols, polyhydric alcohols, ketones, esters, ethers, nitriles, aromatic compounds, and alkyl chlorides. The lower alcohols are not particularly limited, and examples thereof include methanol, ethanol, and absolute ethanol. The polyhydric alcohols are not particularly limited, and examples thereof include propylene glycol and 1,3-butylene glycol. The ketones are not particularly limited, and examples thereof include acetone and formic acid. The esters are not particularly limited, and examples thereof include ethyl acetate. The ethers are not particularly limited, and examples thereof include diethyl ether and dioxane. The nitriles are not particularly limited, and examples thereof include acetonitrile. The aromatic compounds are not particularly limited, and examples thereof include benzene, toluene, and xylene. The alkyl chlorides are not particularly limited, and examples thereof include chloroform.

As the above-described extraction solvent, one kind of solvent may be used, or two or more kinds of solvents may be used in combination, for example. Furthermore, the extraction solvent may be a mixed solvent of the aqueous solvent and the organic solvent, for example. The mixed solvent is not particularly limited, and examples thereof include lower alcohol aqueous solutions such as ethanol aqueous solutions. The proportion of the organic solvent in the mixed solvent is not particularly limited, and is, for example, about 1 vol% to about 99 vol%.

The solvent extraction can be carried out by, for example, providing any of the above-described parts or the entire plant as a raw material and immersing the raw material in the extraction solvent. The raw material may be subjected to treatments such as, for example, washing, drying, and pulverizing, prior to the immersion. When there are two or more kinds of raw materials, each of the raw materials may be subjected to an extraction treatment separately, or a mixture of the two or more kinds of raw materials may be subjected to an extraction treatment. In the former case, for example, the resultant extracts may be mixed together to provide a mixed extract. The proportions of the respective extracts in the mixed extract are not particularly limited, and may be equal proportions (weights), for example. In the latter case, for example, the proportions of the respective raw materials in the mixture are not particularly limited, and may be equal proportions (weights), for example.

The ratio between the raw material and the extraction solvent used in the extraction is not particularly limited. For example, with respect to 100 g (dry weight) of the raw material, there may be 1 to 1000 L of the extraction solvent, preferably 1 to 100 L of the extraction solvent. The immersion time is not particularly limited, and can be set as appropriate depending on, for example, the kinds, the amounts, and the like of the raw material and the extraction solvent. Specifically, in the case where 100 g of the raw material is immersed in 10 L of the extraction solvent, the immersion time preferably is about 0.5 hours or longer, more preferably about 0.5 to about 24 hours, for example.

The temperature of the extraction solvent at the time of extraction is not particularly limited, and may be room temperature, equal to or higher than room temperature, or equal to or lower than room temperature. When the extraction solvent is an aqueous solvent, the extraction treatment preferably is hot water extraction, for example. In the case of the hot water extraction, the temperature of the aqueous solvent is not particularly limited, and may be, for example, 30°C or higher, preferably 50°C to 100°C. Furthermore, the treatment time in the hot water extraction is not particularly limited, and can be set as appropriate depending on, for example, the kind and the amount of the raw material, the amount of the aqueous solvent, and the like. Specifically, when extraction from 100 g (dry weight) of the raw material is performed using 10 L of the aqueous solvent, the treatment time preferably is about 0.5 hours or longer, more preferably about 0.5 to about 24 hours.

After the extraction treatment, the obtained extract may be subjected to a purification treatment or the like, for example. The purification treatment is not particularly limited, and examples thereof include known methods such as a distillation treatment, a filtration treatment, a chromatography treatment, and a drying treatment.

The form of the extract is not particularly limited. For example, the extract may be in the form of liquid, paste, powder, or the like. The form of the extract can be selected as appropriate depending on the form of the AGEs-degrading agent to be described below.

The AGEs-degrading agent may contain only the above-described extract(s), or it may be a composition containing the above-described extract(s) and other components. Examples of the components include various kinds of additives such as the above-described OPH activity enhancer, an excipient, a binding agent, a lubricant, a disintegrant, an absorption promoter, an emulsifying agent, a stabilizing agent, and an antiseptic agent. The blended amounts of the various kinds of additives in the AGEs-degrading agent are not particularly limited, and can be set as appropriate.

The form of the AGEs-degrading agent is not particularly limited. For example, the AGEs-degrading agent may be in the form of solid, gel, liquid, or the like. Specific examples of the form of the AGEs-degrading agent include powder, microgranule, granule, tablet, coated tablet, capsule, troche, eye drop, liquid, patch, lotion, and ointment.

The AGEs-degrading agent used in the present invention may be administered in the form of a composition obtained by mixing it with components of another article, e.g., a food such as a common food or a nutritional supplement, a cosmetic, a quasi-drug, or a pharmaceutical. The blended amount of the AGEs-degrading agent in the composition is not particularly limited, and can be set as appropriate depending on, for example, the kind of the article, subjects to which the composition is administered, and the like.

The AGEs-degrading agent used in the present invention may be used either in vitro or in vivo, for example. In the former case, a subject to which the AGEs-degrading agent is administered may be a cell, a tissue, or the like, for example. The cell or the tissue may be, for example, the one isolated from a living organism or the one cultured after being isolated.

In the latter case, the subject to which the AGEs-degrading agent is administered is a living organism. By administering the AGEs-degrading agent to a living organism, it is possible to degrade AGEs present in the living organism. Thus, the AGEs-degrading agent can be utilized in, for example, a method for treating or preventing diseases, aging of tissues, and hypofunction of tissues caused by AGEs, as will be described below. The living organism is not particularly limited, and may be a human or a non-human animal, for example. Examples of the non-human animal include: nonhuman mammals such as monkeys, cows, pigs, dogs, and cats; birds such as chickens; and fish and shellfish. The administration method is not particularly limited, and examples thereof include oral administration and parenteral administration. Examples of the parenteral administration include transdermal administration and injections such as intravenous injection.

The blended amount of the AGEs-degrading agent in the composition is not particularly limited, and can be set as appropriate, as described above. The dose of the AGEs-degrading agent is not particularly limited, and can be set as appropriate depending on, for example, the animal species, the age, and the like. When a healthy adult orally takes the AGEs-degrading agent, the amount of the extract (solid content) taken preferably is about 5 to about 2000 mg more preferably about 25 to about 1000 mg, for example. The AGEs-degrading agent used in the present invention also can be referred to as a therapeutic agent for treating the above-described diseases, aging of tissues, and/or hypofunction of tissues, and the term therapeutic agent also encompasses a prophylactic agent, for example.

Next, the method for degrading AGEs according to the present invention includes the step of: degrading AGEs in a sample by adding the AGEs-degrading agent as defined by the claims to the sample, as described above. The above description as to the AGEs-degrading agent used in the present invention also applies to the degrading method of the present invention, for example.

### Anti-aging agent and anti-aging method

The anti-aging agent used in the present invention contains the AGEs-degrading agent used in the present invention. The anti-aging agent used in the present invention is characterized in that it contains the AGEs-degrading agent, and other configurations are not particularly limited. The above description as to the AGEs-degrading agent used in the present invention also applies to the anti-aging agent, for example.

The non-therapeutic anti-aging method of the present invention includes the step of administering the AGEs-degradingn agent as defined by the claims. The above descriptions as to the AGEs-degrading agent and AGEs-degrading method of the present invention also apply to the anti-aging method of the present invention, for example.

### Prevention/treatment method

The AGEs-degrading agent that contains at least one kind of plant extract obtained from a plant selected from the group consiting of peanut, knotweed and leaf lettuce as claimed is also for use in the prevention or treatment of diseases, aging of tissues, and hypofunction of tissues caused by AGEs

Examples of the diseases include diabetes complications, Alzheimer's disease, cataracts, arteriosclerosis, osteoporosis, rheumatism, and osteoarthropathy. Examples of the tissues include skin, nerves, kidneys, blood vessels, retinas, crystalline lens, bones, joints, brain tissues, livers, and erythrocytes.

### EXAMPLES

Next, examples of the present invention will be described. It is to be noted, however, that the present invention is by no means limited by the following examples.

### [Example 1]

In the present example, AGEs-degrading activities of plant extracts were evaluated with 1-pheny1-1,2-propanedione (PPD) as an index.

### Preparation of extract samples of plants

Samples of plants shown below were provided. Among these plant samples, undried plant samples were cut with reference to Shokuhin Seibun Hyou 2012 (Tables of Food Composition 2012, Kagawa Nutrition University Publishing Division), and the thus-obtained cut pieces were dried at 65°C for 20 hours using a dryer (Taiki Sangyo food dryer Petit Mini, TAIKISANGYO CO., LTD.). Then, the plant samples provided in the dried form and the plant samples subjected to the above drying treatment were turned to powder by pulverization with a Labo Milser (LM-PLUS, OSAKA CHEMICAL Co., Ltd.). Thereafter, 3.75 g of each of the thus-obtained powder samples was suspended in 150 mL of distilled water at 80°C, and subjected to extraction in a water bath set at 80°C for 1 hour. Thereafter, the mixture was centrifuged, and the supernatant was obtained. The supernatants obtained in this manner were used as extract samples.

| | |
|---|---|
| peanut: | unheated seeds |
| rosemary: | leaves and stems |
| knotweed: | Plumules |
| leaf lettuce: | Leaves |
| basil: | leaves and stems |
| thyme: | leaves and stems |
| spearmint: | leaves and stems |
| lemon balm: | leaves and stems |
| perilla: | Leaves |
| rhubarb: | Petioles |

### Measurement of AGEs-degrading activities of extract samples

The AGEs-degrading activity was evaluated by measuring the cleavage reaction of crosslinks in AGEs. Specifically, the evaluation was carried out in the following manner on the basis of the method described in Vasan et al. (Vasan S, et al., An agent cleaving glucose-derived protein crosslinks in vitro and in vivo. 1996. Nature; 382: pp. 275-278).

As a model substance for crosslinks in AGEs, 1-phenyl-1,2-propanedione (PPD) was used. A degradation reaction was caused by each extract sample with PPD as a substrate. More specifically, a reaction solution having the following composition was obtained by mixing the components in an Eppendorf tube, and the resultant mixture was incubated at 37°C for 8 hours (n = 3). After the incubation, 200 µL of 2 mol/L hydrochloric acid was added to the reaction solution to terminate the reaction. The mixture was centrifuged at 10,000 rpm for 2 minutes, and the supernatant was collected. The amount of benzoic acid released by the degradation of PPD in the supernatant was measured by HPLC carried out under the following conditions. Also, the amount of benzoic acid inherently contained in the extract sample was measured by HPLC carried out under the same conditions. Furthermore, as a positive control regarding the cleavage of crosslinks, the amount of released benzoic acid was measured in the same manner, except that 500 µL of 10 mmol/L phenacylthiazolium bromide (PTB) was used instead of the extract sample.

| **(Composition of Reaction Solution)** | |
|---|---|
| 200 mmol/L phosphate buffer solution | 400 µL |
| 10 mmol/L PPD | 100 µL |
| Extract sample | 500 µL |
| Total | 1000 µL |

| **(HPLC Conditions)** | |
|---|---|
| Column: | Shim-pack FC-ODS/75 × 4.6 mm I.D. |
| | (Shimadzu GLC Ltd.) |
| Eluent: | 2 mmol/L EDTA·2Na-containing 0.2% acetic acid/CAN |
| | (volume ratio: 70/30) |

| **(HPLC Conditions)** | |
|---|---|
| Flow rate: | 1.0 mL/min |
| Detection: | UV 254 nm |
| Injection volume: | 50 µL |

Then, from the amount of benzoic acid in the reaction solution after the reaction, the amount of benzoic acid in the extract sample was subtracted, thereby calculating the molar amount (B) of benzoic acid produced by the degradation of PPD in the reaction solution after the reaction. Next, the thus-calculated molar amount (B) of benzoic acid in the reaction solution after the reaction was divided with the molar amount (P) of PPD in the reaction solution before the reaction. The thus-determined value (B/P) was set as an AGEs crosslink cleavage ratio. On the other hand, regarding the positive control, the value (Bc/P) was determined by dividing the molar amount (Bc) of benzoic acid in the reaction solution after the reaction with the molar amount (P) of PPD in the reaction solution before the reaction, and this value was set as an AGEs crosslink cleavage ratio in the positive control. Then, assuming that the AGEs crosslink cleavage ratio in the positive control is 100%, the relative value (%) of the AGEs crosslink cleavage ratio regarding each extract sample was determined as relative activity.

The results thereof are shown in Table 1 below. As can be seen from Table 1, in the example where the extract samples were added, high AGEs degrading activities were exhibited. Thus, it can be said that each of the plant extracts can degrade and remove AGEs.

**[TABLE 1]**

| **Sample** | **AGEs crosslink cleavage ratio** | **Relative activity** |
|---|---|---|
| | **Mean ± SD (%)** | **Mean ± SD (%)** |
| Peanut | 25.0 ± 2.5 | 65.3 ± 6.4 |
| Rosemary | 24.7 ± 1.7 | 64.5 ± 4.5 |
| Knotweed | 22.8 ± 0.4 | 59.5 ± 1.2 |
| Leaf lettuce | 22.6 ± 1.7 | 59.0 ± 4.4 |
| Basil | 19.4 ± 2.5 | 50.8 ± 6.6 |
| Lemon balm | 18.2 ± 2.7 | 47.7 ± 7.0 |
| Thyme | 15.2 ± 1.9 | 39.7 ± 5.0 |
| Spearmint | 13.7 ± 1.1 | 35.9 ± 2.9 |
| Perilla | 5.6 ± 0.1 | 14.5 ± 0.3 |
| Rhubarb (with skin) | 5.4 ± 0.1 | 14.0 ± 0.2 |
| PTB | 38.2 ± 0.7 | 100.0 |

### [Example 2]

In the present example, the antioxidative activities of plant extracts were evaluated.

### Preparation of plant extract samples

From plants shown in Table 2 below, powder samples were provided and extract samples were prepared in the same manner as in Example 1.

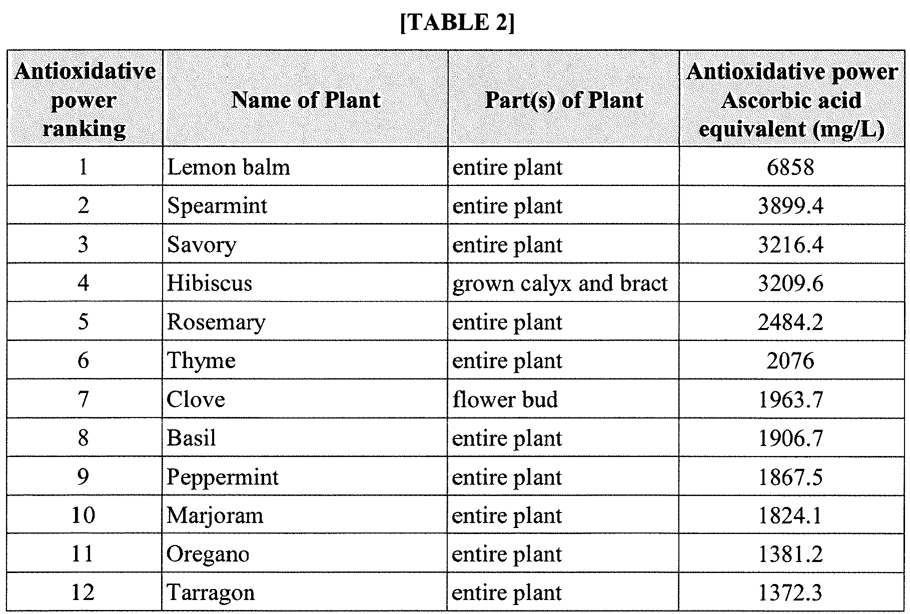

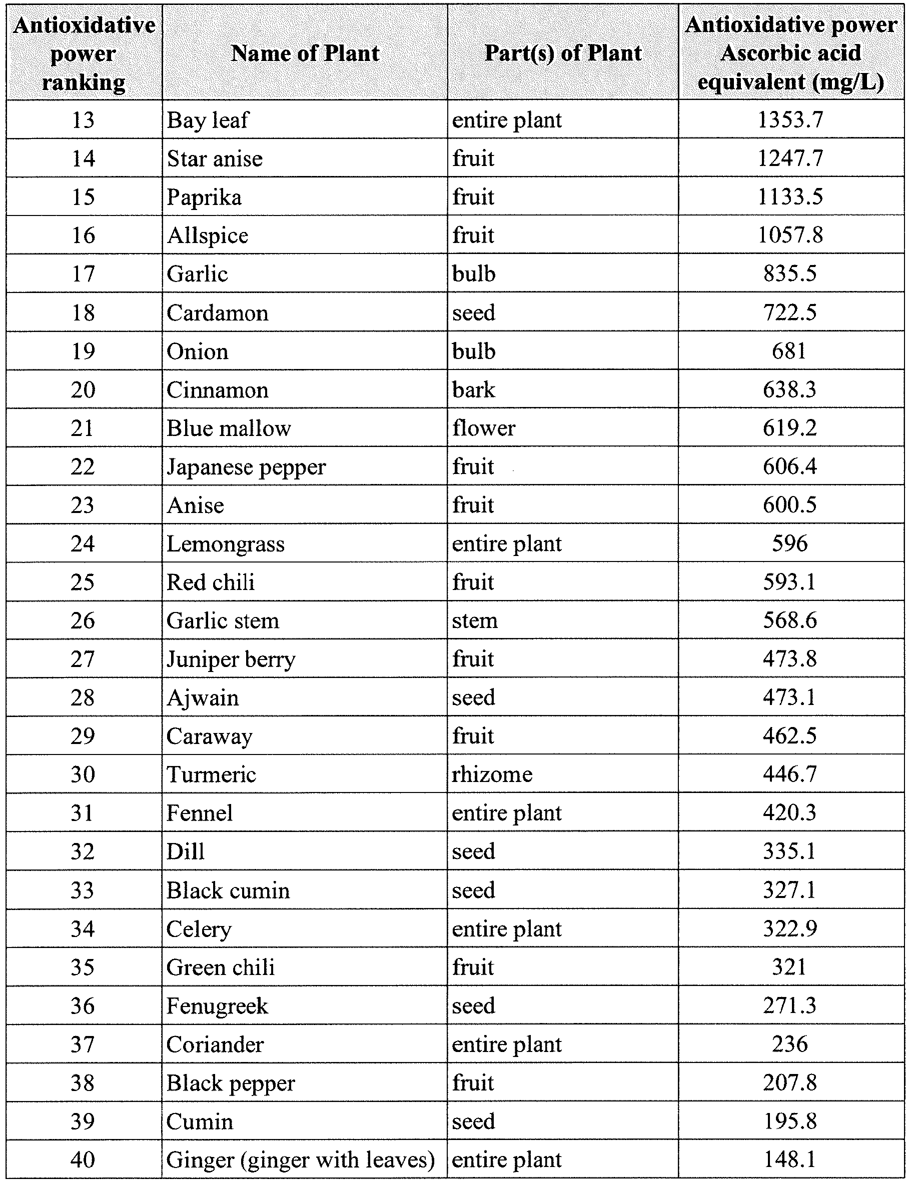

### Measurement of antioxidative power of each extract sample

For the measurement of the antioxidative power, SPOTCHEM (trademark) i-Pack Oxystress Test (trademark) manufactured by ARKRAY, Inc. was used. In a reaction solution containing a thiocyanic acid compound and iron (III) ions, a red complex is formed by the thiocyanic acid compound and the iron (III) ions. Thus, the reaction solution turns red. When this reaction solution further contains a reducing substance, the iron (III) ions forming the complex are reduced to iron (II) ions owing to the reducing action (antioxidative effect) of the reducing substance. As a result, the red color of the reaction solution disappears. On this account, the antioxidative power of each sample was calculated by adding the extract sample to the reaction solution showing red and measuring the change in absorbance.

Specifically, first, 90 µL of thiocyanate-containing SPOTCHEM reagent r1 and 36 µL of iron (III) chloride-containing SPOTCHEM reagent r2 were mixed together. In 126 µL of the thus-obtained mixed solution, the above-described red complex was formed. Then, to 126 µL of the mixed solution, 18 µL of each of the extract samples described in the above item (1) was added. The antioxidative power (µmol/L) of this mixed solution was calculated by measuring the absorbance at 465 nm with the SPOTCHEM. On the other hand, the absorbance measurement was carried out in the same manner except that ascorbic acid as a standard reagent was used instead of the extract sample, and a calibration curve showing the correlation between the ascorbic acid concentration and the absorbance was prepared. Then, on the basis of the absorbance obtained when the extract sample was used and the calibration curve, the antioxidative power of the extract sample was converted to the ascorbic acid equivalent (mg/L).

The results thereof also are shown in Table 2 above. As can be seen from Table 2, all the extracts exhibited antioxidative powers, and in particular, lemon balm, spearmint, savory, hibiscus, rosemary, thyme, clove, and basil exhibited high antioxidative powers. These results demonstrate that, when the AGEs-degrading agent used in the present invention also contains any of these plant extracts, the AGEs-degrading agent can have not only AGEs-degrading ability but also antioxidative power.

While the present invention has been described above with reference to illustrative embodiments, the present invention is by no means limited thereto.

This application claims priority from: Japanese Patent Application No. 2014-108121 filed on May 26, 2014 and Japanese Patent Application No. 2015-104009 filed on May 21, 2015.

### Industrial Applicability

According to the AGEs-degrading agent used in the present invention, it is possible to degrade AGEs. Therefore, it can be for use in treating and preventing diseases, aging of tissues, and hypofunction of tissues caused by AGEs produced in vivo, for example. Furthermore, the present invention is excellent in terms of safety, because it uses plant extracts that have been used since ancient times. Also, the present invention is excellent in terms of productivity, because the plant extracts can be obtained in large quantities.

## Claims

1. A non-therapeutic anti-aging method comprising the step of administering an AGEs-degrading agent, the agent comprising:
at least one kind of plant extract obtained from a plant selected from the group consisting of peanut, knotweed and leaf lettuce.

2. The non-therapeutic anti-aging method according to claim 1, wherein the AGEs-degrading agent comprises two or more kinds of the plant extracts.

3. The non-therapeutic anti-aging method according to claim 2, wherein the two or more kinds of the plant extracts comprise, among the plant extracts obtained from plants selected from the group consisting of peanut, knotweed and leaf lettuce, also serves to enhance activity of oxidized protein hydrolase.

4. The non-therapeutic anti-aging method according to any one of claims 1 to 3, wherein the agent further comprises an oxidized protein hydrolase activity enhancer,
wherein the oxidized protein hydrolase activity enhancer comprises at least one kind of plant extract obtained from a plant selected from the group consisting of water chestnut, stevia, Chrysanthemum, savory, mugwort, and chestnut.

5. The non-therapeutic anti-aging method according to any one of claims 1 to 4, wherein the agent further comprises an antioxidant,
wherein the antioxidant comprises at least one kind of plant extract obtained from a plant selected from the group consisting of lemon balm, savory, hibiscus, rosemary, thyme, clove, peppermint, marjoram, oregano, tarragon, bay leaf, star anise, paprika, allspice, garlic, cardamon, onion, cinnamon, blue mallow, Japanese pepper, anise, lemongrass, chili pepper, juniper berry, ajwain, caraway, turmeric, fennel, dill, black cumin, celery, fenugreek, coriander, pepper, cumin, and ginger.

6. A formulation comprising an AGEs-degrading agent for use in preventing or treating diseases and hypofunction of tissues caused by AGEs,
the AGEs-degrading agent comprising at least one kind of plant extract obtained from a plant selected from the group consisting of peanut, knotweed and leaf lettuce.

7. The formulation for use according to claim 6, wherein the AGEs-degrading agent comprises two or more kinds of the plant extracts.

8. The formulation for use according to claim 7, wherein the two or more kinds of the plant extracts comprise, among the plant extracts obtained from plants selected from the group consisting of peanut, knotweed, leaf lettuce also serves to enhance activity of oxidized protein hydrolase.

9. The formulation for use according to any one of claims 6 to 8, wherein the agent further comprises an oxidized protein hydrolase activity enhancer,
wherein the oxidized protein hydrolase activity enhancer comprises at least one kind of plant extract obtained from a plant selected from the group consisting of water chestnut, stevia, Chrysanthemum, savory, mugwort, and chestnut.

10. The formulation for use according to any one of claims 6 to 9, wherein the agent further comprises an antioxidant,
wherein the antioxidant comprises at least one kind of plant extract obtained from a plant selected from the group consisting of lemon balm, savory, hibiscus, rosemary, thyme, clove, peppermint, marjoram, oregano, tarragon, bay leaf, star anise, paprika, allspice, garlic, cardamon, onion, cinnamon, blue mallow, Japanese pepper, anise, lemongrass, chili pepper, juniper berry, ajwain, caraway, turmeric, fennel, dill, black cumin, celery, fenugreek, coriander, pepper, cumin, and ginger.

11. A method for degrading AGEs, the method comprising the step of
degrading AGEs in a sample by adding an AGEs-degrading agent, the agent comprising at least one kind of plant extract obtained from a plant selected from the group consisting of peanut, knotweed and leaf lettuce.

12. The method according to claim 11, wherein the AGEs-degrading agent comprises two or more kinds of the plant extracts.

13. The method according to claim 12, wherein the two or more kinds of the plant extracts comprise, among the plant extracts obtained from plants selected from the group consisting of peanut, rosemary, knotweed, leaf lettuce, lemon balm, and perilla, at least one kind of plant extract obtained from a plant selected from the group consisting of rosemary, thyme, and lemon balm, which, preferably, also serves to enhance activity of oxidized protein hydrolase.

14. The method according to any one of claims 11 to 13, wherein the agent further comprises an oxidized protein hydrolase activity enhancer,
wherein the oxidized protein hydrolase activity enhancer comprises at least one kind of plant extract obtained from a plant selected from the group consisting of water chestnut, stevia, Chrysanthemum, savory, mugwort, and chestnut.

15. The method according to any one of claims 11 to 14, wherein the agent further comprises an antioxidant,
wherein the antioxidant comprises at least one kind of plant extract obtained from a plant selected from the group consisting of lemon balm, savory, hibiscus, rosemary, thyme, clove, peppermint, marjoram, oregano, tarragon, bay leaf, star anise, paprika, allspice, garlic, cardamon, onion, cinnamon, blue mallow, Japanese pepper, anise, lemongrass, chili pepper, juniper berry, ajwain, caraway, turmeric, fennel, dill, black cumin, celery, fenugreek, coriander, pepper, cumin, and ginger.

## Patentansprüche

1. Nicht-therapeutisches Anti-Aging-Verfahren, das den Schritt der Verabreichung eines AGEs-degradierenden Wirkstoffs umfasst, der Wirkstoff umfassend:
mindestens eine Art von Pflanzenextrakt erhalten aus einer Pflanze, die aus der Gruppe bestehend aus Erdnuss, Knöterich und Blattsalat ausgewählt ist.

2. Nicht-therapeutisches Anti-Aging-Verfahren nach Anspruch 1, wobei der AGEs-degradierende Wirkstoff zwei oder mehr Arten der Pflanzenextrakte umfasst.

3. Nicht-therapeutisches Anti-Aging-Verfahren nach Anspruch 2, wobei die zwei oder mehr Arten der Pflanzenextrakte umfassen, unter den Pflanzenextrakten erhalten aus Pflanzen, die aus der Gruppe bestehend aus Erdnuss, Knöterich und Blattsalat ausgewählt sind, auch dazu dienen die Aktivität der oxidierten Proteinhydrolase zu erhöhen.

4. Nicht-therapeutisches Anti-Aging-Verfahren nach einem der Ansprüche 1 bis 3, wobei der Wirkstoff ferner einen Aktivitätsverstärker der oxidierten Proteinhydrolase umfasst,
wobei der Aktivitätsverstärker der oxidierten Proteinhydrolase mindestens eine Art von Pflanzenextrakt erhalten aus einer Pflanze umfasst, die aus der Gruppe bestehend aus Wasserkastanie, Stevia, Chrysantheme, Bohnenkraut, Beifuß, und Kastanie ausgewählt ist.

5. Nicht-therapeutisches Anti-Aging-Verfahren nach einem der Ansprüche 1 bis 4, wobei der Wirkstoff ferner ein Antioxidans umfasst,
wobei das Antioxidans mindestens eine Art von Pflanzenextrakt erhalten aus einer Pflanze umfasst, die aus der Gruppe bestehend aus Zitronenmelisse, Bohnenkraut, Hibiskus, Rosmarin, Thymian, Nelke, Pfefferminze, Majoran, Oregano, Estragon, Lorbeer, Sternanis, Paprika, Piment, Knoblauch, Kardamon, Zwiebel, Zimt, blaue Malve, japanischer Pfeffer, Anis, Zitronengras, Chilipfeffer, Wacholderbeere, Ajowan, Kümmel, Kurkuma, Fenchel, Dill, Schwarzkümmel, Sellerie, Bockshornklee, Koriander, Pfeffer, Kumin, und Ingwer ausgewählt ist.

6. Formulierung, die einen AGEs-degradierenden Wirkstoff umfasst, zur Anwendung bei der Prävention oder Behandlung von Krankheiten und Hypofunktion von Geweben verursacht durch AGEs,
der AGEs-degradierende Wirkstoff umfassend mindestens eine Art von Pflanzenextrakt erhalten aus einer Pflanze, die aus der Gruppe bestehend aus Erdnuss, Knöterich und Blattsalat ausgewählt ist.

7. Formulierung zur Anwendung nach Anspruch 6, wobei der AGEs-degradierende Wirkstoff zwei oder mehr Arten der Pflanzenextrakte umfasst.

8. Formulierung zur Anwendung nach Anspruch 7, wobei die zwei oder mehr Arten der Pflanzenextrakte umfassen, unter den Pflanzenextrakten erhalten aus Pflanzen, die aus der Gruppe bestehend aus Erdnuss, Knöterich, Blattsalat ausgewählt sind, auch dazu dienen die Aktivität der oxidierten Proteinhydrolase zu erhöhen.

9. Formulierung zur Anwendung nach einem der Ansprüche 6 bis 8, wobei der Wirkstoff ferner einen Aktivitätsverstärker der oxidierten Proteinhydrolase umfasst,
wobei der Aktivitätsverstärker der oxidierten Proteinhydrolase mindestens eine Art von Pflanzenextrakt erhalten aus einer Pflanze umfasst, die aus der Gruppe bestehend aus Wasserkastanie, Stevia, Chrysantheme, Bohnenkraut, Beifuß, und Kastanie ausgewählt ist.

10. Formulierung zur Anwendung nach einem der Ansprüche 6 bis 9, wobei der Wirkstoff ferner ein Antioxidans umfasst,
wobei das Antioxidans mindestens eine Art von Pflanzenextrakt erhalten aus einer Pflanze umfasst, die aus der Gruppe bestehend aus Zitronenmelisse, Bohnenkraut, Hibiskus, Rosmarin, Thymian, Nelke, Pfefferminze, Majoran, Oregano, Estragon, Lorbeer, Sternanis, Paprika, Piment, Knoblauch, Kardamon, Zwiebel, Zimt, blaue Malve, japanischer Pfeffer, Anis, Zitronengras, Chilipfeffer, Wacholderbeere, Ajowan, Kümmel, Kurkuma, Fenchel, Dill, Schwarzkümmel, Sellerie, Bockshornklee, Koriander, Pfeffer, Kumin, und Ingwer ausgewählt ist.

11. Verfahren zum Degradieren von AGEs, das Verfahren umfassend den Schritt des Degradierens von AGEs in einer Probe durch Zugabe eines AGEs-degradierenden Wirkstoffs, der Wirkstoff umfassend mindestens eine Art von Pflanzenextrakt erhalten aus einer Pflanze, die aus der Gruppe bestehend aus Erdnuss, Knöterich und Blattsalat ausgewählt ist.

12. Verfahren nach Anspruch 11, wobei der AGEs-degradierende Wirkstoff zwei oder mehr Arten der Pflanzenextrakte umfasst.

13. Verfahren nach Anspruch 12, wobei die zwei oder mehr Arten der Pflanzenextrakte umfassen, unter den Pflanzenextrakten erhalten aus Pflanzen, die aus der Gruppe bestehend aus Erdnuss, Rosmarin, Knöterich, Blattsalat, Zitronenmelisse, und Perilla ausgewählt sind, mindestens eine Art von Pflanzenextrakt erhalten aus einer Pflanze, die aus der Gruppe bestehend aus Rosmarin, Thymian, und Zitronenmelisse ausgewählt ist, das, bevorzugt, auch dazu dient die Aktivität der oxidierten Proteinhydrolase zu erhöhen.

14. Verfahren nach einem der Ansprüche 11 bis 13, wobei der Wirkstoff ferner einen Aktivitätsverstärker der oxidierten Proteinhydrolase umfasst,
wobei der Aktivitätsverstärker der oxidierten Proteinhydrolase mindestens eine Art von Pflanzenextrakt erhalten aus einer Pflanze umfasst, die aus der Gruppe bestehend aus Wasserkastanie, Stevia, Chrysantheme, Bohnenkraut, Beifuß, und Kastanie ausgewählt ist.

15. Verfahren nach einem der Ansprüche 11 bis 14, wobei der Wirkstoff ferner ein Antioxidans umfasst,
wobei das Antioxidans mindestens eine Art von Pflanzenextrakt erhalten aus einer Pflanze umfasst, die aus der Gruppe bestehend aus Zitronenmelisse, Bohnenkraut, Hibiskus, Rosmarin, Thymian, Nelke, Pfefferminze, Majoran, Oregano, Estragon, Lorbeer, Sternanis, Paprika, Piment, Knoblauch, Kardamon, Zwiebel, Zimt, blaue Malve, japanischer Pfeffer, Anis, Zitronengras, Chilipfeffer, Wacholderbeere, Ajowan, Kümmel, Kurkuma, Fenchel, Dill, Schwarzkümmel, Sellerie, Bockshornklee, Koriander, Pfeffer, Kumin, und Ingwer ausgewählt ist.

## Revendications

1. Procédé antivieillissement non thérapeutique comprenant l'étape d'administration d'un agent de dégradation d'AGE, l'agent comprenant :
au moins un type d'extrait de plante obtenu à partir d'une plante choisie dans le groupe constitué de l'arachide, de la renouée et de la laitue à couper.

2. Procédé antivieillissement non thérapeutique selon la revendication 1, dans lequel l'agent de dégradation d'AGE comprend deux types ou plus des extraits de plante.

3. Procédé antivieillissement non thérapeutique selon la revendication 2, dans lequel les deux types ou plus des extraits de plante comprennent, parmi les extraits de plante obtenus à partir de plantes choisies dans le groupe constitué de l'arachide, de la renouée et de la laitue à coupe, sert également à amplifier l'activité d'une protéine hydrolase oxydée.

4. Procédé antivieillissement non thérapeutique selon l'une quelconque des revendications 1 à 3, dans lequel l'agent comprend en outre un amplificateur d'activité de protéine hydrolase oxydée,
dans lequel l'amplificateur d'activité de protéine hydrolase oxydée comprend au moins un type d'extrait de plante obtenu à partir d'une plante choisie dans le groupe constitué de la châtaigne d'eau, de la stévia, du chrysanthème, de la sarriette, de l'armoise et de la châtaigne.

5. Procédé antivieillissement non thérapeutique selon l'une quelconque des revendications 1 à 4, dans lequel l'agent comprend en outre un antioxydant,
dans lequel l'antioxydant comprend au moins un type d'extrait de plante obtenu à partir d'une plante choisie dans le groupe constitué de la citronnelle, de la sarriette, de l'hibiscus, du romarin, du thym, du clou de girofle, de la menthe poivrée, de la marjolaine, de l'origan, de l'estragon, de la feuille de laurier, de l'anis étoilé, du paprika, du piment de la Jamaïque, de l'ail, de la cardamome, de l'oignon, de la cannelle, de la mauve bleue, du poivre du Japon, de l'anis, du lemon-grass, du poivre de Cayenne, de la baie de genièvre, de l'ajowan, du carvi commun, du curcuma, du fenouil, de l'aneth, du cumin noir, du céleri, du fenugrec, de la coriandre, du poivre, du cumin et du gingembre.

6. Formulation comprenant un agent de dégradation d'AGE pour une utilisation dans la prévention ou le traitement de maladies et d'une hypofonction de tissus causées par des AGE,
l'agent de dégradation d'AGE comprenant au moins un type d'extrait de plante obtenu à partir d'une plante choisie dans le groupe constitué de l'arachide, de la renouée et de la laitue à couper.

7. Formulation pour une utilisation selon la revendication 6, dans laquelle l'agent de dégradation d'AGE comprend deux types ou plus des extraits de plante.

8. Formulation pour une utilisation selon la revendication 7, dans laquelle les deux types ou plus des extraits de plante comprennent, parmi les extraits de plante obtenus à partir de plantes choisies dans le groupe constitué de l'arachide, de la renouée, de la laitue à coupe, sert également à amplifier l'activité d'une protéine hydrolase oxydée.

9. Formulation pour une utilisation selon l'une quelconque des revendications 6 à 8, dans laquelle l'agent comprend en outre un amplificateur d'activité de protéine hydrolase oxydée,
dans laquelle l'amplificateur d'activité de protéine hydrolase oxydée comprend au moins un type d'extrait de plante obtenu à partir d'une plante choisie dans le groupe constitué de la châtaigne d'eau, de la stévia, du chrysanthème, de la sarriette, de l'armoise et de la châtaigne.

10. Formulation pour une utilisation selon l'une quelconque des revendications 6 à 9, dans laquelle l'agent comprend en outre un antioxydant,
dans laquelle l'antioxydant comprend au moins un type d'extrait de plante obtenu à partir d'une plante choisie dans le groupe constitué de la citronnelle, de la sarriette, de l'hibiscus, du romarin, du thym, du clou de girofle, de la menthe poivrée, de la marjolaine, de l'origan, de l'estragon, de la feuille de laurier, de l'anis étoilé, du paprika, du piment de la Jamaïque, de l'ail, de la cardamome, de l'oignon, de la cannelle, de la mauve bleue, du poivre du Japon, de l'anis, du lemon-grass, du poivre de Cayenne, de la baie de genièvre, de l'ajowan, du carvi commun, du curcuma, du fenouil, de l'aneth, du cumin noir, du céleri, du fenugrec, de la coriandre, du poivre, du cumin et du gingembre.

11. Procédé de dégradation d'AGE, le procédé comprenant l'étape de
dégradation d'AGE dans un échantillon par ajout d'un agent de dégradation d'AGE, l'agent comprenant au moins un type d'extrait de plante obtenu à partir d'une plante choisie dans le groupe constitué de l'arachide, de la renouée et de la laitue à couper.

12. Procédé selon la revendication 11, dans lequel l'agent de dégradation d'AGE comprend deux types ou plus des extraits de plante.

13. Procédé selon la revendication 12, dans lequel les deux types ou plus des extraits de plante comprennent, parmi les extraits de plante obtenus à partir de plantes choisies dans le groupe constitué de l'arachide, du romarin, de la renouée, de la laitue à couper, de la citronnelle et du perilla, au moins un type d'extrait de plante obtenu à partir d'une plante choisie dans le groupe constitué du romarin, du thym et de la citronnelle, qui, de préférence, sert également à amplifier l'activité d'une protéine hydrolase oxydée.

14. Procédé selon l'une quelconque des revendications 11 à 13, dans lequel l'agent comprend en outre un amplificateur d'activité de protéine hydrolase oxydée,
dans lequel l'amplificateur d'activité de protéine hydrolase oxydée comprend au moins un type d'extrait de plante obtenu à partir d'une plante choisie dans le groupe constitué de la châtaigne d'eau, de la stévia, du chrysanthème, de la sarriette, de l'armoise et de la châtaigne.

15. Procédé selon l'une quelconque des revendications 11 à 14, dans lequel l'agent comprend en outre un antioxydant,
dans lequel l'antioxydant comprend au moins un type d'extrait de plante obtenu à partir d'une plante choisie dans le groupe constitué de la citronnelle, de la sarriette, de l'hibiscus, du romarin, du thym, du clou de girofle, de la menthe poivrée, de la marjolaine, de l'origan, de l'estragon, de la feuille de laurier, de l'anis étoilé, du paprika, du piment de la Jamaïque, de l'ail, de la cardamome, de l'oignon, de la cannelle, de la mauve bleue, du poivre du Japon, de l'anis, du lemon-grass, du poivre de Cayenne, de la baie de genièvre, de l'ajowan, du carvi commun, du curcuma, du fenouil, de l'aneth, du cumin noir, du céleri, du fenugrec, de la coriandre, du poivre, du cumin et du gingembre.
